# EUROPÄISCHE PATENTANMELDUNG

(11) **EP 1 239 281 A2**
(43) Veröffentlichungstag der Anmeldung: **11.09.2002**
(21) Anmeldenummer: 02090081.7
(22) Anmeldetag: 28.02.2002
(51) Int. Cl.: G01N 31/22, G01N 33/28

(54) **Schnelltest zur Bestimmung saurer oder alkalischer Bestandteile in Ölen**

(30) Priorität: 09.03.2001 DE 10111390
(71) Anmelder: Schmack Biogas GmbH, 93133 Burglengenfeld (DE)
(72) Erfinder: Nusko, Robert, Dr., 93109 Wiesent (DE)
(74) Vertreter: Bast, Tim, Dr.

(57) **Zusammenfassung**

Die Erfindung betrifft einen Schnelltest zur Bestimmung des Zustandes von Ölen.

Der Schnelltest besteht aus einem transparenten Aufbewahrungsmittel, in dem sich ein spezielles wässriges Reagens befindet. Nach Zugabe einer definierten Ölmenge in das Röhrchen und Schütteln ergibt sich bei der Überschreitung eines einstellbaren Schwellenwertes aus einer Änderung des pH-Wertes im Reagens durch Extraktion von "sauren" bzw. "basischen" Bestandteilen aus dem Öl in das wässrige Reagens ein Farbumschlag durch den zugesetzten Säure-Base Indikator.

Durch definierte pH-Wert Einstellung des Reagens, und Zugabe unterschiedlicher Indikatorfarbstoffe kann der Schnelltest auf verschiedene Öltypen und -mengen und auf verschiedene Schwellenwerte eingestellt werden. Durch Verwendung mehrerer Indikatorfarbstoffe in einem Röhrchen oder durch Anwendung unterschiedlich eingestellter Testsets kann der Zustand eines Öls näher bestimmt werden. Die Erfindung erlaubt eine einfache Abschätzung des "sauren" bzw. "alkalischen" Zustandes von Ölen und erübrigt aufwendige Laboruntersuchungen bezüglich des i-pH-Wertes, des TAN-, TBN-Wertes oder der Neutralisationszahl.

## Beschreibung

### Anwendungsgebiet

Die Erfindung betrifft einen Schnelltest zur Bestimmung des Zustandes von Ölen, insbesondere Motorölen für Verbrennungsmotoren.

### Stand der Technik

Der Zustand von Motorölen ist entscheidend für einen reibungslosen Betrieb der Motoren. Zur Kontrolle der Ölqualität werden eine Reihe von Untersuchungen durchgeführt. Dabei werden die Werte für die Belastung der Öle mit Partikeln, mit Verschleißmetallen oder sonstigen Verunreinigungen, der Wassergehalt, die Viskosität, die Total Base Number (TBN), die Total Acid Number (TAN), die Neutralisationszahl (DIN 51 369, 1981) oder der i-pH-Wert (Anfangs pH-Wert nach IP 177/83 bei 0 mL Zugabe von Kaliumhydroxid) bestimmt. Gerade die TAN oder auch der i-pH-Wert geben Aufschluss über den Anteil an "sauren" Bestandteilen im Öl. Bei Gasmotorenölen sind diese Parameter, wegen der häufig in Gasen vorkommenden sauren Gase von besonderer Bedeutung. Säuren können den Werkstoff, mit dem das Öl in Berührung kommt angreifen. Der "saure" Zustand von Ölen wird standardmäßig durch Farbindikator-Titration (DIN 51 558, Teil 1, 1979, Teil 2, 1990), durch potentiometrische Titration (IP 177/83, 1988), oder durch Säure-Base-Titration im nicht wässrigen System (ISO 660-1983; ASTM D664-89) bestimmt. Mit Hilfe von speziellen Reagenzien ist die Bestimmung des "sauren" Zustandes von Ölen durch eine einfache pH-Messung nach Extraktion (US 5,366,898; US 5,800,782) oder im Zweiphasensystem bestehend aus dem Öl und einem speziellen, wasserhaltigen Reagens möglich (US 6,027,940; US 6,174,731).

Nicht nur Motoröle werden auf ihren Zustand hin untersucht. Auch in anderen Bereichen werden die eingesetzten Öle auf Qualität, Herkunft und Alterung geprüft. Beispielhaft seien die Qualitätssicherung in der Lebensmittelindustrie und die regelmäßige Überprüfung von Elektroisolierölen aufgeführt.

### Nachteile des Standes der Technik

Die Bestimmung des "sauren" Zustandes von Motorölen nach den bekannten Verfahren ist nur in einem ausgerüsteten Labor durch ausgebildetes Fachpersonal durchführbar.

Meist sind für die Bestimmung giftige und brennbare Lösungsmittel (Toluol, Diethylether, Methylisobutylketon,...) notwendig.

Mit Hilfe spezieller Reagenzien ist es möglich die Säuren aus dem Öl in diese Reagenzien zu extrahieren (US 6,027,940) und anschließend den Säurewert des Öls durch eine pH-Messung im Zweiphasensystem zu bestimmen. Auch hier ist eine Laborausrüstung notwendig. Die Bestimmung kann kaum direkt vor Ort gleich nach der Ölprobennahme durchgeführt werden.

Alle zur Bestimmung des "sauren" Zustandes von Öl eingesetzten Verfahren sind zeitaufwendig und vergleichsweise kostenintensiv. Es steht somit keine Methode zur Verfügung, die eine schnelle Abschätzung des Säurezustandes von Ölen mit Hilfe eines einfach zu handhabenden Schnelltests ermöglicht.

### Aufgabe der Erfindung

Der "Säurezustand" von Ölen ist ein wichtiger und charakteristischer Parameter für die Qualität des Öles.

Gerade bei Gasmotorenölen besteht eine erhöhte Gefahr der Übersäuerung durch saure Gase. Durch schwankende Gaszusammensetzungen kann es zu schnellen Veränderungen des "Säurezustandes" im Motoröl kommen. Zur Vermeidung von motorschädigenden Korrosionen oder säurebedingter Veränderungen der Schmiereigenschaften des Öls muss das Öl kontrolliert und bei Übersäuerung ausgetauscht werden. Üblicherweise wird für die Bestimmung des Qualitätszustandes von Motorölen der sog. TAN - Wert durch Titration oder der Anfangs pH-Wert (i-pH-Wert) durch pH-Wertmessung nach Extraktion ermittelt. Eine Aufgabe der Erfindung ist es mit Hilfe eines Schnelltestes den "Säurezustand" von Motorölen schnell auf einfache Art und Weise direkt vor Ort und ohne Laborgeräte abschätzen zu können, bzw. eine Grenzwertüberschreitung anzuzeigen.

Ziel der vorliegenden Erfindung ist die rasche, einfache und frühzeitige Identifizierung einer schädlichen Versäuerung von Öl, bzw. die Identifizierung eines bestimmten Öls. Alkalisch wirkende Ölverunreinigungen können ebenfalls nachgewiesen werden.

Darüber hinaus kann der erfindungsgemäße Schnelltest zur Qualitätskontrolle von Isolierölen eingesetzt werden. Durch Alterung, Wasseraufnahme, usw. verändert sich der "saure" Charakter dieser Öle - diese Veränderung kann mit dem beschriebenem Test erfasst werden.

Eine weitere Aufgabe der Erfindung ist die Qualitätssicherung bzw. Identifizierung von Ölen. Mittels des Tests können schnell und einfach die sauren bzw. alkalischen Bestandteile eines Öles überprüft werden. Diese Werte sind häufig charakteristisch für bestimmte Öle. Die Überwachung mit Hilfe des erfindungsgemäßen Schnelltests kann zumindest ein Baustein in einer prozessinternen Qualitätssicherungskette sein.

### Kurze Zusammenfassung der Erfindung

Die vorliegende Erfindung beschreibt ein Verfahren zur Bestimmung saurer oder alkalischer Bestandteile in Ölen, dadurch gekennzeichnet, dass das Verfahren die folgenden Schritte enthält
- Entnehmen einer Ölprobe
- Vermischen der Ölprobe mit einer wässrigen Reagenzienmischung, wobei die Reagenzienmischung einen Säure/Base-Indikator-Farbstoff enthält
- Herbeiführen oder Abwarten des Ausbildens der Phasentrennung in eine Ölphase und eine wässrige Phase
- Bestimmung der Farbveränderung der wässrigen Phase
- Beurteilung des Anteils von sauren oder basischen Anteilen in der Ölprobe basierend auf der Farbe der wässrigen Phase

Die vorliegende Erfindung beschreibt außerdem ein Verfahren zur Bestimmung saurer oder alkalischer Bestandteile in Ölen, dadurch gekennzeichnet dass die wässrige Reagenzienmischung zusätzlich eine grenzflächenaktive Substanz enthält.

Die vorliegende Erfindung beschreibt außerdem ein Verfahren zur Bestimmung saurer oder alkalischer Bestandteile in Ölen, dadurch gekennzeichnet dass es sich bei der grenzflächenaktiven Substanz um ein nichtionisches Tensid handelt.

Die vorliegende Erfindung beschreibt außerdem ein Verfahren zur Bestimmung saurer oder alkalischer Bestandteile in Ölen, dadurch gekennzeichnet dass das nichtionische Tensid in einem Konzentrationsbereich von 0,001 bis 0,1 Gew.-% der wässrigen Reagenzienmischung eingesetzt wird, wobei eine Konzentration von 0,01 Gew.-% bevorzugt wird.

Die vorliegende Erfindung beschreibt außerdem ein Verfahren zur Bestimmung saurer oder alkalischer Bestandteile in Ölen, dadurch gekennzeichnet dass der Säure/Base - Indikator Farbstoff in einer Konzentration eingesetzt wird, die eine Bestimmung der Farbveränderung mit dem Auge ermöglichen.

Die vorliegende Erfindung beschreibt außerdem ein Verfahren zur Bestimmung saurer oder alkalischer Bestandteile in Ölen, dadurch gekennzeichnet dass die wässrige Reagenzienmischung zwei oder mehr Säure/Base - Indikator Farbstoffe enthält werden, bzw. dass zur Verbesserung der Auswertbarkeit der Farbveränderung der / des Indikatorfarbstoffes unter den Einsatzbedingungen stabile Farbstoffe zugegeben werden, die die Farbe der / des Indikatorfarbstoffes überlagern.

Die vorliegende Erfindung beschreibt außerdem ein Verfahren zur Bestimmung saurer oder alkalischer Bestandteile in Ölen, dadurch gekennzeichnet dass die wässrige Reagenzienmischung zusätzlich ein oder mehrere Leitsalze enthält, wobei bevorzugt Alkalimetallhalogenide aus der Gruppe KCl, NaCl, LiCl, KBr, und wobei bevorzugt die Leitsalze der wässrigen Reagenzienmischung in einer Gesamtkonzentration von zwischen 0,1 und 3 Mol pro Liter wässrige Reagenzienmischung zugesetzt werden.

Die vorliegende Erfindung beschreibt außerdem ein Verfahren zur Bestimmung saurer oder alkalischer Bestandteile in Ölen, dadurch gekennzeichnet dass der pH-Wert der wässrigen Reagenzienmischung mittels Säuren bzw. Basen auf einen für den gewünschten Umschlag geeigneten Wert eingestellt ist.

Die vorliegende Erfindung beschreibt außerdem ein Verfahren zur Bestimmung saurer oder alkalischer Bestandteile in Ölen, dadurch gekennzeichnet dass die pH-Wert Einstellung mit Alkalimetallsalzlauge erfolgt, wobei die Verwendung von NaOH bzw. KOH bevorzugt wird.

Die vorliegende Erfindung beschreibt außerdem ein Verfahren zur Bestimmung saurer oder alkalischer Bestandteile in Ölen, dadurch gekennzeichnet dass der pH-Wert auf einen Wert zwischen 7 und 9 eingestellt ist, und bevorzugt auf einen pH-Wert zwischen 7,5 und 8,2 eingestellt ist.

Die vorliegende Erfindung beschreibt außerdem ein Verfahren zur Bestimmung saurer oder alkalischer Bestandteile in Ölen, dadurch gekennzeichnet dass die wässrige Reagenzienmischung unter Schutzgasatmosphäre produziert und in Aufbewahrungsmittel abgefüllt wird, wobei als Schutzgas die Verwendung von CO₂freier Luft, Stickstoff oder Edelgas bevorzugt wird.

Die vorliegende Erfindung beschreibt außerdem eine wässrige Reagenzienmischung zur Bestimmung saurer Bestandteile in Ölen, dadurch gekennzeichnet dass die Reagenzienmischung zum Einsatz nach einem der in den vorgenannten Ansprüchen 1 bis 11 beschriebenen Verfahren bestimmt ist.

Die vorliegende Erfindung beschreibt außerdem eine Reagenzienmischung zur Herstellung der vorgenannten wässrigen Reagenzienmischung.

Die vorliegende Erfindung beschreibt außerdem eine Vorrichtung zur Bestimmung saurer Bestandteile in Ölen, dadurch gekennzeichnet dass eine der vorgenannten Reagenzienmischungen enthalten ist.

Die vorliegende Erfindung beschreibt außerdem eine Vorrichtung zur Bestimmung saurer Bestandteile in Ölen, dadurch gekennzeichnet dass die Vorrichtung zusätzlich ein oder mehrere die wässrige Reagenzienmischung enthaltende, transparente, wieder verschließbare Aufbewahrungsmittel, sowie ein oder mehrere zur Entnahme einer Ölprobe mit einem definierten Volumen geeignetes Mittel enthält.

Die vorliegende Erfindung beschreibt außerdem eine Vorrichtung zur Bestimmung saurer Bestandteile in Ölen, dadurch gekennzeichnet dass die Vorrichtung zusätzlich Farbkarten oder Vergleichlösungen zur Durchführung des Bestimmung der Farbe der wässrigen Phase enthält.

Die vorliegende Erfindung beschreibt außerdem eine Vorrichtung zur halbquantitativen Bestimmung saurer Bestandteile, dadurch gekennzeichnet dass die Vorrichtung zwei oder mehrere, auf unterschiedliche pH-Werte eingestellte Vorrichtungen vom einem der oben beschriebenen Typen enthält.

### Vorteile der Erfindung

Bei der Erfindung handelt es sich um einen Schnelltest zur Bestimmung der sauren bzw. alkalischen Bestandteile von Ölen. Der Schnelltest besteht im wesentlichen aus einem transparenten Gefäß, das ein spezielles Reagens enthält. Das Reagens zeigt nach der Zugabe einer definierten Ölmenge und kurzem Schütteln eine Farbänderung, wenn das Öl übersäuert ist bzw. zu viele alkalische Bestandteile enthält. Mit Hilfe dieses Schnelltestes kann eine mögliche Übersäuerung bzw. eine Säure/Base Puffergrenze von Motorölen schnell und ohne Benutzung von Laborgeräten ermittelt werden.

Der Test ist einfach durchzuführen und kann von angelerntem Personal direkt nach der Ölprobennahme vor Ort angewendet werden. Für das Erkennen einer Motorölübersäuerung, bzw. einer Überschreitung der Säure/Base Puffergrenze von Ölen sind somit keine aufwendigen Laboruntersuchungen notwendig.

Das Reagens enthält keine giftigen oder brennbaren Lösungsmittel.

Zur Ermittlung des "Säurezustandes" von Motorölen steht hiermit ein kostengünstiger Schnelltest zur Verfügung, der es erlaubt eine motorschädigende Übersäuerung von Motorölen frühzeitig zu erkennen.

Der Test ist handlich - er kann ohne Aufwand mitgeführt und direkt an der Anlage eingesetzt werden.

Der Test kann zum kostengünstigen Screening vieler Proben eingesetzt werden.

Das Ergebnis der Messung steht wenige Minuten nach der Probennahme zur Verfügung.

Der Versand von Proben an ein Labor ist überflüssig - oder nur in Grenzfällen nötig.

Die Messung kann vom Betreiber einer Anlage jederzeit durchgeführt werden.

Die erforderliche Ölprobenmenge (ca. 1 mL) ist sehr gering. Damit werden für den Test keine nennenswerten Ölmengen verbraucht.

Der Test ist einfach und sicher in der Handhabung und einfach und sicher in der Auswertung - es ist kein Fachpersonal erforderlich.

Durch die iterative Durchführung unterschiedlich abgestimmter Tests ist zumindest eine halbquantitative Bestimmung der TAN oder des i-pH-Wertes mittels eines Schnelltests möglich.

### Beschreibung der Erfindung

Der Schnelltest besteht aus einem Reagens in einem transparenten Aufbewahrungsmittel wie zum Beispiel einem Röhrchen mit Stopfen. Nach Zugabe einer aus dem Öl entnommenen Probe definierten Volumens zum Reagens und kurzem Schütteln wird das Röhrchen abgestellt. Befindet sich das getestete Öl in einem übersäuerten Zustand bzw. ist die Säure/Base Puffergrenze überschritten kann nach der sich innerhalb weniger Minuten einstellenden Phasentrennung eine Farbänderung des Reagens in der wässrigen Phase registriert werden. Alternativ kann die Phasentrennung auch beschleunigt durch Zentrifugieren hergestellt werden. Nach dem Gebrauch werden die Schnellteströhrchen verworfen.

Das wässrige Reagens dient zur Extraktion der sauren bzw. alkalischen Bestandteile aus der Ölprobe in die wässrige Phase. Der Zusatz eines Tensides ermöglicht die Bildung einer feinen Öl / Wasser Emulsion beim Schütteln. In dieser Emulsion liegt eine große Wasser / Öl Phasengrenze vor. Austauschprozesse zwischen Öl und der wässrigen Phase werden damit erleichtert bzw. stark beschleunigt. Beim Schütteln gelangen somit in der Ölprobe vorhandene saure bzw. alkalische Bestandteile innerhalb kurzer Zeit in das wässrige Reagens bzw. verändern dort die vorliegende Protonenkonzentration.

Der Zusatz von Leitsalzen zu dem wässrigen Reagens ermöglicht einer bessere Phasentrennung zwischen Öl- und wässriger Phase nach der Extraktion der sauren Bestandteile aus der Ölprobe in das wässrige Reagens. Geeignete Leitsalze wie zum Beispiel KCl, NaCl, LiCl, KBr, und ähnliche sind im Stand der Technik bekannt.

Der Zusatz eines Säure-Base-Indikators ermöglicht eine binäre Anzeige einer pH-Grenzwertüberschreitung im wässrigen Reagens nach der Extraktion von sauren Bestandteilen aus der Ölprobe. Geeignete Säure-Base-Indikatorfarbstoffe wie die in den Beispielen aufgeführten und andere mehr sind im Stand der Technik bekannt.

Durch die Zugabe definierter Mengen an Basen bzw. Säuren kann das wässrige Reagens auf einen definierten pH-Wert eingestellt werden. Mit dieser pH-Einstellung, der gewählten Mengenverhältnisse Ölprobe / Reagens und der Auswahl des Indikatorfarbstoffs kann der Schnelltest auf den gewünschten Umschlag angepasst werden. Diese Anpassung ist für verschiedene Öle separat vorzunehmen. So kann der Test z.B. auf die Anzeige der Überschreitung bestimmter TAN - Werte bzw. i-pH-Werte bzw. Neutralisationszahlen eingestellt werden (je nach analytischem Problem korrelieren dieses Werte meist - eine Abstimmung der Anzeige des Schnelltests auf die genannten Parameter ist für verschiedene Einsatzgebiete erforderlich). Durch die Variation zugegebener Säure- / Basemenge und dem Einsatz verschiedener Indikatorfarbstoffe kann ein ganzes Set an Tests geschaffen werden, dessen sequentielle Anwendung die Eingrenzung des "sauren" Zustandes eines Öls zulässt. Ähnliche Aussagen lassen sich durch die Verwendung mehrerer Indikatorfarbstoffe in einem Röhrchen gewinnen - dieses Mischindikatorprinzip ist aus vielen pH-Teststreifen bekannt. Des weiteren eignet sich die Verwendung von Mischindikatoren zur Fertigung eines universellen Tests, mit dessen Hilfe verschiedene Öle, die unterschiedliche Arbeits-pH-Werte aufweisen getestet werden können. Außerdem ist durch die Verwendung von Mischindikatoren die Fertigung eines Tests möglich, mit dessen Hilfe sowohl Übersäuerung, als auch alkalische Verunreinigungen nachgewiesen werden können.

Zur Stabilisierung des Reagens können die Kunststoffröhrchen unter CO₂ freier Luft, Stickstoff, Edelgasen oder anderen geeigneten Schutzgasen abgefüllt werden.

Das für saure Ölbestandteile Beschriebene gilt natürlich sinngemäß auch für alkalische Bestandteile. Auch hierfür sind die geeigneten Reagenzien wie zum Beispiel Indikatorfarbstoffe im Stand der Technik bekannt.

### Detaillierte Beschreibung der Erfindung

Für eine bevorzugte Ausführung des erfindungsgemäßen Schnelltests werden einfache Kunststoffröhrchen mit Lamellenstopfen (erhältlich zum Beispiel von Semadeni (Europe) AG, Düsseldorf, Deutschland als Tube PS, 2 mL, 11 * 42 mm; Stopfen PE-LD 8,5 - 10 mm) verwendet. Bei der Herstellung des wässrigen Reagens werden 0,1 Gew.-% eines nichtionischen Tensides, z.B. Triton X-100 (Sigma Aldrich als Fluka No 93418) in einer 0,5 bis 1,0 M Lösung eines Leitsalzes gelöst. Als Leitsalz eignen sich Kaliumchlorid, Kaliumnitrat, Natriumchlorid, oder ein anderes Alkalimetallsalz. Als Indikator für die Anzeige eines Farbumschlages beim Überschreiten einer bestimmten Konzentration der aus dem Öl in das Reagens extrahierten sauren Bestandteile wird ein Säure-Base-Indikator zugegeben. Die Auswahl des Indikators wird durch den gewünschten Umschlagsbereich und das zu untersuchende Öl definiert (z.B. Phenolphthalein, Bromthymolblau, Bromkresolpurpur, Bromkresolgrün, und ähnliche). Durch die Zugabe einer definierten Menge einer Base wird der pH-Wert des Reagens so eingestellt, dass der verwendete Indikator in der alkalischen Form vorliegt und frisches, säurefreies Motoröl bzw. Motoröl mit geringen Säuregehalten keinen Farbumschlag bewirken. Als Base wird eine 0,1 M Natronlauge verwendet. Das Reagens wird zur Vermeidung von pH-Wert Schwankungen durch Eintrag von CO₂ aus der Luft unter CO₂ - freier Luft produziert und in das Kunststoffröhrchen abgefüllt.

### Ausführungsbeispiele

### Beispiel 1

### Herstellung des Reagens:

- Als Leitsalz wird Kaliumchlorid (KCl, Riedel de Haen, p.a. 99,5 %) verwendet.
- Zu 190 g einer 0,75 Mol/L KCl Lösung werden 0,2 g Triton X-100 (Fluka No 93418) gegeben.
- Anschließend werden 0,01 g des Säure-Base-Indikators Bromkresolpurpur Natriumsalz (Fluka, Standard Fluka) zugegeben.
- Mit 0,1 Mol/L Natronlauge (Fluka, Reag. Ph. Eur. No 35263) wird der pH-Wert des Reagens auf 7,9 bis 8,0 eingestellt. Das Reagens ist bei diesem pH-Wert Wert violett.
- Anschließend wird mit 0,75 Mol/L KCl Lösung auf 200 g aufgefüllt.
- 1 mL des so hergestellten Reagens werden unter CO₂ freier Luft in ein Plastikröhrchen (Fa. Semadeni: Tube: PS, 2 mL, 11 * 42 mm) abgefüllt. Das Röhrchen wird mit einem Lamellenstopfen (Fa. Semadeni Stopfen: PE-LD 8,5 - 10 mm) verschlossen.

Das Reagens ist nach dem Abfüllen in die Kunststoffröhrchen gebrauchsfertig.

Das nach dem beschriebenen Verfahren hergestellte Reagens ist abgestimmt auf die Unterschreitung eines i-pH-Wertes von 4,2 bei gebrauchtem Gasmotorenöl Esso Estor PX 40 der Firma Esso Deutschland GmbH.

Es werden Gasmotorenöle dieses Typs nach 0, 600 und 800 Betriebsstunden in einem Biogasmotor getestet. Hierzu werden jeweils 0,5 mL Öl in eines der o.g. Teströhrchen gegeben und etwa 30 s intensiv geschüttelt. Das Teströhrchen wird anschließend auf den Kopf (Deckel) gestellt. Nach etwa 3 min haben sich die wässrige und die ölige Phase wieder getrennt. In der wässrigen Phase kann bei einer Übersäuerung des Öl ein Farbwechsel von violett nach gelb registriert werden.

Der Farbumschlag von violett nach gelb erfolgt erstmals nach einer Betriebszeit von 800 Stunden. In parallel durchgeführten Messungen wurde der i-pH-Wert dieses Öls auf 4,0 bestimmt. Die i-pH-Werte der anderen Ölproben, die keinen Farbumschlag des Schnelltests bewirkt haben lagen jeweils über 4,5.

### Beispiel 2

Herstellung des Reagens wie in Beispiel 1 beschrieben, ohne Verwendung von Kaliumchlorid als Leitsalz. Als Lösungsmittel wird entionisiertes Wasser verwendet.

### Beobachtung:

Die Phasentrennung zwischen der wässrigen Reagensphase und dem Öl nach dem Schütteln erfolgt langsamer als in Beispiel 1.

Die Durchführung der in Beispiel 1 genannten Tests an gebrauchtem Gasmotorenöl führt zu denselben Ergebnissen, wie in Beispiel 1.

### Beispiel 3

Herstellung des Reagens wie in Beispiel 1 beschrieben. Anstatt Triton X-100 werden 0,2 g Tagat TO (Fa. Goldschmidt, Nr. 200194) verwendet.

Die Durchführung der in Beispiel 1 genannten Tests an gebrauchtem Gasmotorenöl führt zu denselben Ergebnissen, wie in Beispiel 1. Phasentrennung und Farbumschlag sind den in Beispiel 1 genannten vergleichbar.

## Patentansprüche

1. Verfahren zur Bestimmung saurer oder alkalischer Bestandteile in Ölen, **dadurch gekennzeichnet, dass** das Verfahren die folgenden Schritte enthält
- Entnehmen einer Ölprobe
- Vermischen der Ölprobe mit einer wässrigen Reagenzienmischung, wobei die Reagenzienmischung einen Säure/Base-Indikator-Farbstoff enthält
- Herbeiführen oder Abwarten des Ausbildens der Phasentrennung in eine Ölphase und eine wässrige Phase
- Bestimmung der Farbveränderung der wässrigen Phase
- Beurteilung des Anteils von sauren oder basischen Anteilen in der Ölprobe basierend auf der Farbe der wässrigen Phase

2. Verfahren nach dem vorgenannten Anspruch 1, **dadurch gekennzeichnet dass** die wässrige Reagenzienmischung zusätzlich eine grenzflächenaktive Substanz enthält.

3. Verfahren nach dem vorgenannten Anspruch 2, **dadurch gekennzeichnet dass** es sich bei der grenzflächenaktiven Substanz um ein nichtionisches Tensid handelt.

4. Verfahren nach dem vorgenannten Anspruch 3, **dadurch gekennzeichnet dass** das nichtionische Tensid in einem Konzentrationsbereich von 0,001 bis 0,1 Gew.-% der wässrigen Reagenzienmischung eingesetzt wird, wobei eine Konzentration von 0,01 Gew.-% bevorzugt wird.

5. Verfahren nach einem der vorgenannten Ansprüche 1 bis 4, **dadurch gekennzeichnet dass**
der Säure/Base - Indikator Farbstoff in einer Konzentration eingesetzt wird, die eine Bestimmung der Farbveränderung mit dem Auge ermöglichen.

6. Verfahren nach einem der vorgenannten Ansprüche 1 bis 5, **dadurch gekennzeichnet dass**
die wässrige Reagenzienmischung zwei oder mehr Säure/Base - Indikator Farbstoffe enthält.

7. Verfahren nach einem der vorgenannten Ansprüche 1 bis 6, **dadurch gekennzeichnet dass**
die wässrige Reagenzienmischung zusätzlich ein oder mehrere Leitsalze enthält, wobei bevorzugt Alkalimetallhalogenide aus der Gruppe KCl, NaCl, LiCl, KBr, und bevorzugt die Leitsalze der wässrigen Reagenzienmischung in einer Gesamtkonzentration von zwischen 0,1 und 3 Mol pro Liter wässrige Reagenzienmischung zugesetzt werden.

8. Verfahren nach einem der vorgenannten Ansprüche 1 bis 7, **dadurch gekennzeichnet dass**
der pH-Wert der wässrigen Reagenzienmischung mittels Säuren bzw. Basen auf einen für den gewünschten Umschlag geeigneten Wert eingestellt ist.

9. Verfahren nach dem vorgenannten Anspruch 8, **dadurch gekennzeichnet dass** die pH-Werteinstellung mit Alkalimetallsalzlauge erfolgt, wobei die Verwendung von NaOH bzw. KOH bevorzugt wird.

10. Verfahren nach einem der vorgenannten Ansprüche 8 bis 9, **dadurch gekennzeichnet dass**
der pH-Wert auf einen Wert zwischen 7 und 9 eingestellt ist, und bevorzugt wobei auf einen pH-Werte zwischen 7,5 und 8,2 eingestellt ist.

11. Verfahren nach einem der vorgenannten Ansprüche 1 bis 10, **dadurch gekennzeichnet dass**
die wässrige Reagenzienmischung unter Schutzgasatmosphäre produziert und in Aufbewahrungsmittel abgefüllt wird, wobei als Schutzgas die Verwendung von CO₂-freier Luft, Stickstoff oder Edelgas bevorzugt wird.

12. Wässrige Reagenzienmischung zur Bestimmung saurer Bestandteile in Ölen, **dadurch gekennzeichnet dass**
die Reagenzienmischung zum Einsatz nach einem der in den vorgenannten Ansprüchen 1 bis 11 beschriebenen Verfahren bestimmt ist.

13. Reagenzienmischung zur Herstellung der in dem vorgenannten Anspruch 12 beschriebenen wässrigen Reagenzienmischung.

14. Vorrichtung zur Bestimmung saurer Bestandteile in Ölen, **dadurch gekennzeichnet dass**
den vorgenannten Ansprüchen 12 oder 13 entsprechende Reagenzienmischungen enthalten sind.

15. Vorrichtung nach dem vorgenannten Anspruch 14, **dadurch gekennzeichnet dass** die Vorrichtung zusätzlich ein oder mehrere die wässrige Reagenzienmischung enthaltende, transparente, wieder verschließbare Aufbewahrungsmittel, sowie ein oder mehrere zur Entnahme einer Ölprobe mit einem definierten Volumen geeignetes Mittel enthält.

16. Vorrichtung nach einem der vorgenannten Ansprüche 14 oder 15, **dadurch gekennzeichnet dass**
die Vorrichtung zusätzlich Farbkarten oder Vergleichlösungen zur Durchführung der Bestimmung der Farbe der wässrigen Phase enthält.

17. Vorrichtung zur halbquantitativen Bestimmung saurer Bestandteile, **dadurch gekennzeichnet dass**
die Vorrichtung zwei oder mehrere, auf unterschiedliche pH-Werte eingestellte Vorrichtungen nach einem der vorgenannten Ansprüche 14 bis 16 enthält.
